(19) Europäisches Patentamt
European Patent Office
Office européen des brevets

(11) **EP 4 006 166 A2**

(12) **EUROPEAN PATENT APPLICATION**
published in accordance with Art. 153(4) EPC

(43) Date of publication:
**01.06.2022 Bulletin 2022/22**

(21) Application number: **20859995.1**

(22) Date of filing: **03.11.2020**

(51) International Patent Classification (IPC):
**C12Q 1/68** (2018.01)        **G01N 33/68** (2006.01)
**G01N 33/574** (2006.01)

(52) Cooperative Patent Classification (CPC):
**A61K 38/08; A61K 45/00; A61P 35/00;**
**A61P 35/04; C12Q 1/68; C12Q 1/6886;**
**G01N 33/574; G01N 33/68**

(86) International application number:
**PCT/CN2020/126029**

(87) International publication number:
**WO 2021/043340 (11.03.2021 Gazette 2021/10)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **05.09.2019  CN 201910850263**

(71) Applicant: **Nanjing Anji Biotechnology Co., Ltd.**
**Nanjing, Jiangsu 210038 (CN)**

(72) Inventors:
• **XU, Hanmei**
  **Nanjing, Jiangsu 211100 (CN)**
• **LI, Mengwei**
  **Nanjing, Jiangsu 211100 (CN)**

(74) Representative: **Kramer Barske Schmidtchen**
**Patentanwälte PartG mbB**
**European Patent Attorneys**
**Landsberger Strasse 300**
**80687 München (DE)**

Remarks:
The restoration of the right of priority granted by the
receiving Office is effective before the EPO (R.
49ter.1(a) PCT).

(54)   **TUMOUR MARKER AQUAPORIN 2 PROTEIN AND APPLICATION THEREOF**

(57)    Disclosed are a tumour marker aquaporin 2 protein and an application thereof, belonging to the fields of tumour detection and molecular targeted therapy. The present invention comprises applications of the aquaporin 2 protein in the following functions: (1) as a new tumour marker used for the early diagnosis and prognostic determination of malignant tumours including head and neck squamous cell carcinoma, kidney cancer and prostate cancer; (2) inhibiting tumour cell proliferation; (3) inhibiting tumour cell migration; (4) inhibiting animal transplant tumour model growth. Targeted therapy which uses the aquaporin 2 protein as a biomarker, as in the present invention, provides new ideas for the treatment of malignant tumours such as head and neck squamous cell carcinoma, kidney cancer and prostate cancer.

Fig. 7

EP 4 006 166 A2

**Description**

**TECHNICAL FIELD**

The present invention pertains to the fields of tumor detection and molecular targeted therapy and more specifically, relates to a transmembrane protein AQUAPORIN 2 ("AQP2" for short) and an application thereof.

**BACKGROUND ART**

Tumors are currently the most serious diseases endangering human health. Studies have found that the generation of tumors is a complex process of gradual accumulation of gene mutations, and the development of modern medical technology and molecular biology has brought tumor treatment into the era of individualization and greatly increased the remission rate of tumor treatment. Therefore, finding specific targets is crucial to early diagnosis, treatment and prognosis of tumors and a key bottleneck restricting the clinical efficacy of tumors.

Head and neck cancer includes neck tumors (thyroid tumors, etc.), ENT tumors (larynx cancer, nasopharyngeal cancer, paranasal sinus cancer, etc.) and oral and maxillofacial tumors (tongue cancer, gum cancer, cheek cancer, etc.). More than 90% of head and neck tumors are squamous cell carcinoma. Head and neck squamous cell carcinoma is the sixth most common cancer in the world, with more than 500,000 new cases worldwide each year, and the 5-year survival rate of not more than 40%. At present, the treatment methods still mainly include radiotherapy, chemotherapy and surgery, with poor clinical prognosis. Therefore, studying in depth the pathogenesis of head and neck squamous cell carcinoma and discovering new biomarkers are of great significance for the targeted therapy of head and neck squamous cell carcinoma and the prognosis of patients.

Kidney cancer, also known as renal cell carcinoma, originates from renal tubular epithelial cells and is the most common renal parenchymal malignancy. There are about 208,500 new cases every year in the world, and the incidence in China is about 4.5/100,000. At present, the etiology of kidney cancer is not clear, and most patients with kidney cancer are found not sensitive to radiotherapy and chemotherapy in clinical treatment and mostly relying on surgery. Therefore, improving the accuracy of early diagnosis is helpful for the timely treatment of kidney cancer patients.

Prostate cancer refers to epithelial malignant tumors that occur in the prostate and mainly includes adenocarcinoma (acinar adenocarcinoma), ductal adenocarcinoma, urothelial carcinoma, squamous cell carcinoma and adenosquamous carcinoma. The incidence increases with age and reaches a peak at the age of 70 to 80 years. There are obvious regional and racial differences in the incidence of prostate cancer. According to statistics, the incidence of prostate cancer is the lowest in Chinese and the highest in Europeans. In recent years, with the improvement of living conditions and the prolongation of life expectancy, the incidence of prostate cancer in China has also increased year by year.

Tumor metastasis and invasion are important features of malignant tumors and the main culprits of most tumor recurrences. Studies have found that tumor metastasis and invasion is a continuous dynamic process involving multiple genes, of which proto-oncogenes and cancer suppressor genes play an equally important role. The effects of a large number of proto-oncogenes such as PTEN, MYC, RAS, PIK3CA and AKT1 in malignant tumors including head and neck squamous cell carcinoma have been revealed in depth, while studies on tumor suppressor genes except TP53 have been rarely reported. With the help of bioinformatics methods such as high-throughput screening and big data analysis, the discovery of tumor suppressor genes with important functions is very important for revealing the pathogenesis of tumors and proposing more comprehensive diagnosis and treatment plans.

Aquaporin-2 (AQP2), a member of the aquaporin family, is mainly distributed in the luminal membrane and intracellular vesicles of chief cells of the collecting duct, and is an antidiuretic hormone-sensitive aquaporin. Current studies have found that AQP2 is mainly expressed in kidney tissue and is involved in the pathological processes of diseases such as neurological diabetes insipidus and polycystic kidney disease. However, the expression and functions of AQP2 in tumors have not been reported in the literature. This study discovered for the first time the expression level and potential biological functions of AQP2 in different types of tumors, which is important for the development of the application value of AQP2 in tumor detection and treatment.

**SUMMARY OF THE INVENTION**

To address the existing problem that malignant tumors such as head and neck squamous cell carcinoma do not have closely related biomarkers, the present invention provides a tumor marker AQP2 protein and successfully applied it in tumor detection and treatment. Through bioinformatics methods, clinical tumor samples and biological function experiments, new biomarkers closely related to the occurrence, development and metastasis of head and neck squamous cell carcinoma, kidney cancer and prostate cancer were discovered.

The present invention adopts the following technical solution:

Application of a transmembrane protein in the preparation of tumor treatment drugs or the use as a tumor marker, wherein the marker is transmembrane protein AQP2, and its amino acid sequence is shown in SEQ ID NO.2.

Preferably, tumors that this tumor marker is used to detect include head and neck squamous cell carcinoma, kidney cancer and prostate cancer.

A kit for detecting the expression of the foregoing marker, wherein the detection kit includes a specific primer pair designed for the nucleotide sequence encoding AQP2 (shown in SEQ ID NO. 1).

Preferably, reagents for detecting biomarker expression can be used in tools for prognosis of tumor subjects. The method of prognosis described herein includes: obtaining a test sample from a tumor; determining the expression level of the biomarker in the test sample; and analyzing the expression level to generate a risk score, which can be used to provide a prognosis for the subject. It should be noted that the test samples used in the prognosis are fresh, frozen, or paraffin-fixed and -embedded tissue.

Preferably, the foregoing detection reagents are reagents containing anti-AQP2 protein antibody and can also be composition detection reagents containing anti-AQP2 protein antibody.

The method for detecting the foregoing biomarker, wherein specific primers are designed, a PCR method is used to detect the expression quantity of transmembrane protein AQP2 in tissue cells, and the primer sequences are shown in SEQ ID NO. 3 and SEQ ID NO. 4.

A recombinant vector achieving overexpression of the transmembrane protein AQP2, wherein the recombinant vector can be applied in the preparation of drugs for treating tumors.

Preferably, the recombinant vector is an overexpression plasmid, lentivirus or cell line containing the nucleotide sequence shown in SEQ ID NO. 1 and having the following functions (a1) to (a3):

(a1) inhibiting tumor growth;

(a2) inhibiting proliferation of tumor cells;

(a3) inhibiting migration of tumor cells.

Compared with the prior art, the present invention has the following advantages:

(1) For the first time, it was found that transmembrane protein AQP2 played an important role in tumor diagnosis, prognosis and treatment, and could be used as a tumor marker of head and neck squamous cell carcinoma, kidney cancer and prostate cancer.

(2) The present invention found that the expression levels of transmembrane protein AQP2 in head and neck squamous cell carcinoma cells, kidney cancer cells and prostate cancer cells were significantly lower than that in normal epithelial cells, and AQP2 overexpression could significantly inhibit the proliferation, migration and in vivo tumor growth of head and neck squamous cell carcinoma cells, kidney cancer cells and prostate cancer cells, which demonstrate the importance of AQP2 for tumor growth and metastasis and suggest that AQP2 has the potential as a target for drug design. For example, antitumor substances targeting AQP2 (containing overexpression plasmid vector, lentivirus or transgenic cell line encoding nucleotide) can be used to prepare drugs against head and neck squamous cell carcinoma, kidney cancer and prostate cancer.

(3) The present invention uses GAPDH as an internal reference gene to detect the expression level of AQP2. It is found that the expression quantities of AQP2 protein in head and neck squamous cell carcinoma cells SCC4, kidney cancer cells 786-O, and prostate cancer cells DU145 were significantly reduced, which proves that AQP2 can be used as a new biomarker to diagnose malignant tumors including head and neck squamous cell carcinoma, kidney cancer and prostate cancer.

## BRIEF DESCRIPTION OF THE DRAWINGS

Fig. 1 is the comparison of the expression quantities of AQP2 gene in head and neck squamous cell carcinoma tissue and paracancer tissue of human. The data is from TCGA database;

Fig. 2 is the comparison of the expression quantities of AQP2 gene in papillary cell renal carcinoma tissue and paracancer tissue of human. The data is from TCGA database;

Fig. 3 is the comparison of the expression quantities of AQP2 gene in clear cell renal carcinoma tissue and paracancer tissue of human. The data is from TCGA database;

Fig. 4 is the comparison of the expression quantities of AQP2 gene in chromophobe cell renal carcinoma tissue and paracancer tissue of human. The data is from TCGA database;

Fig. 5 is the comparison of the expression quantities of AQP2 gene in prostate cancer tissue and paracancer tissue of human. The data is from TCGA database;

Fig. 6 is the comparison of the expression quantities of AQP2 gene in three types of tumor cells and normal cells;

Fig. 7 is the comparison of the AQP2 protein expression quantities of AQP2 gene in three types of tumor cells and normal cells;

Fig. 8 is the map of a lentiviral overexpression vector of AQP2;

Fig. 9 shows the effect of overexpression AQP2 on the expression quantities of AQP2 gene and protein in head and neck squamous cell carcinoma cells;

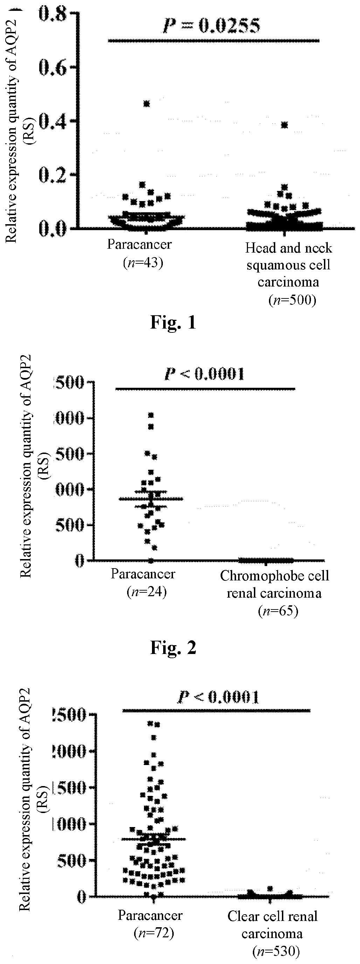

Fig. 1 shows the effect of overexpression AQP2 on the expression quantities of AQP2 gene and protein in kidney cancer cells;

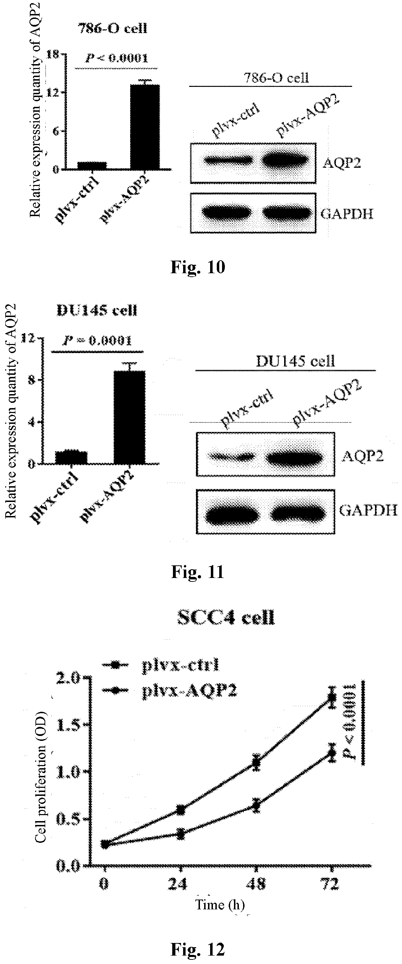

Fig. 11 shows the effect of overexpression AQP2 on the expression quantities of AQP2 gene and protein in prostate cancer cells;

Fig. 12 shows the effect of overexpression AQP2 on the proliferation ability of head and neck squamous cell carcinoma cells SCC4;

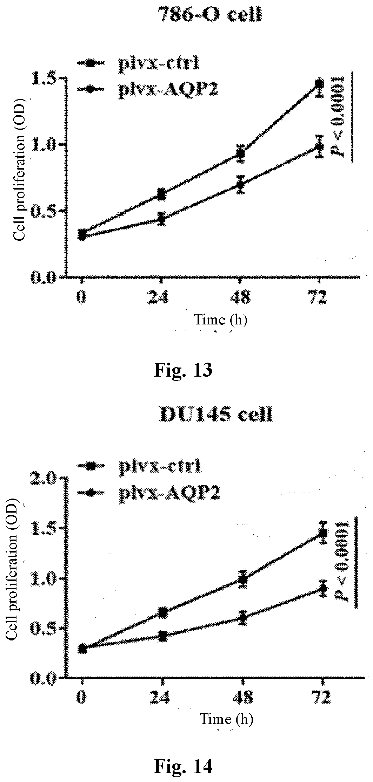

Fig. 13 shows the effect of overexpression AQP2 on the proliferation ability of kidney cancer cells 786-O;

Fig. 14 shows the effect of overexpression AQP2 on the proliferation ability of prostate cancer cells DU145.

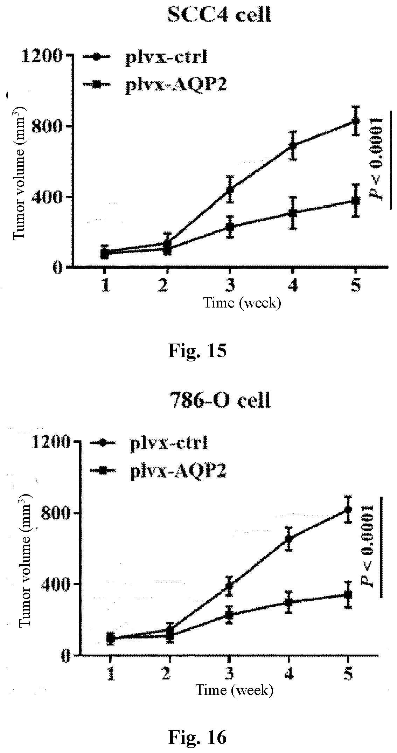

Fig. 15 shows the effect of overexpression AQP2 on the in vivo tumor growth of head and neck squamous cell carcinoma cells SCC4;

Fig. 16 shows the effect of overexpression AQP2 on the in vivo tumor growth of kidney cancer cells 786-O;

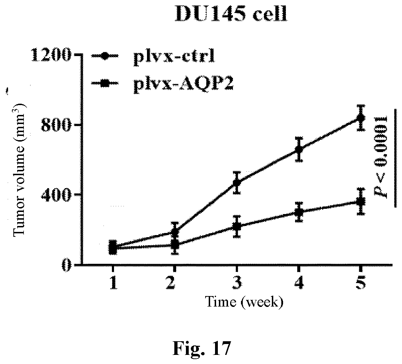

Fig. 17 shows the effect of overexpression AQP2 on the in vivo tumor growth of prostate cancer cells DU145.

## DETAILED DESCRIPTION

The present invention will be further described below in conjunction with specific embodiments.

## Embodiment 1

Expression profile microarray analysis of AQP2 in different human tumor tissues and paracancer tissues
The Cancer Genome Atlas (TCGA) Program was jointly initiated by the National Cancer Institute (NCI) and the National Human Genome Research Institute (NHGRI) of the United States in 2006. It applies the genome analysis technology based on large-scale sequencing to conduct large-scale experiments on 36 types of cancers. The Genome Characterization Center (GCC) of TCGA compares tumors and normal tissues to look for gene mutations, amplifications or deletions associated with each cancer or subtype and help understand the molecular mechanism of cancer and improve the scientific understanding on the molecular basis of cancer pathogenesis.
The whole gene expression profile data and clinical information of 36 tumors and their paracancer tissues were downloaded by the TCGA standard method, R language (3.1.1 version) software was used to filter away the tumor types not containing AQP2 expression information, and AP2 expression was detected in 20 types of tumors.

Table 1. Analysis of expression levels of AQP2 in different tumors in TCGA database

| Tumor type | Sample size | Value $P$ |
|---|---|---|
| Bladder cancer | Tumor (408) Normal (19) | 0.0689 |
| Breast cancer | Tumor (1090) Normal (113) | 0.1059 |
| Cervical squamous cell carcinoma | Tumor (304) Normal (3) | 0.1502 |
| Gallbladder cancer | Tumor (36) Normal (9) | 0.0594 |
| Colon cancer | Tumor (454) Normal (41) | 0.0721 |
| Esophageal cancer | Tumor (161) Normal (11) | 0.0831 |
| Head and neck squamous cell carcinoma | Tumor (500) Normal (44) | **0.0255** |
| Chromophobe carcinoma | Tumor (65) Normal (24) | **<0.0001** |
| Clear cell renal carcinoma | Tumor (530) Normal (72) | **<0.0001** |
| Papillary cell renal carcinoma | Tumor (288) Normal (32) | **<0.0001** |
| Liver cancer | Tumor (371) Normal (50) | 0.0825 |
| Pulmonary adenocarcinoma | Tumor (513) Normal (59) | 0.0613 |
| Pulmonary squamous cell carcinoma | Tumor (501) Normal (49) | 0.126 |
| Pancreatic cancer | Tumor (177) Normal (4) | 0.198 |
| Adrenal carcinoma | Tumor (178) Normal (3) | 0.465 |
| Prostate cancer | Tumor (495) Normal (52) | **<0.0001** |
| Rectal cancer | Tumor (165) Normal (10) | 0.134 |
| Stomach cancer | Tumor (375) Normal (32) | 0.251 |
| Thyroid cancer | Tumor (502) Normal (58) | 0.0624 |
| Endometrial cancer | Tumor (543) Normal (23) | 0.0582 |

Graphpad Prism7 was used to conduct statistical analysis on the expression levels of AQP2 in 20 types of tumor tissues and corresponding paracancer tissues. All data underwent statistical $t$ test, *$P$<0.05 means significant difference, and **P<0.01 means very significant difference.

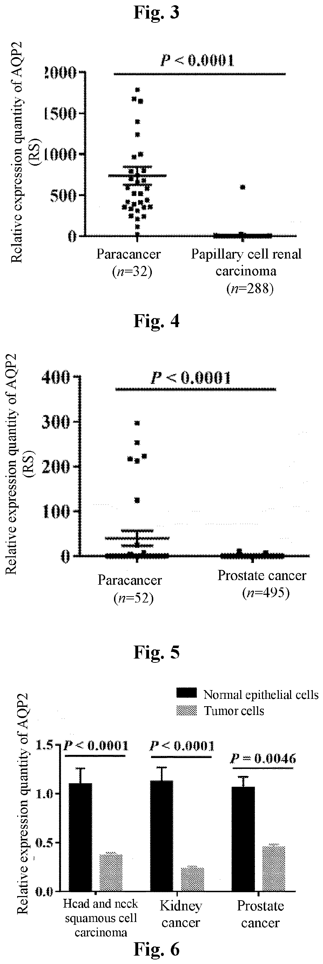

The specific analysis results are shown in Table 1. Compared with the corresponding paracancer tissues, AQP2 showed significant low expression in head and neck squamous cell carcinoma (Fig. 1), three renal carcinoma subtypes (papillary cell renal carcinoma, clear cell renal carcinoma, chromophobe renal carcinoma, Fig. 2 to Fig. 4) and prostate cancer (Fig. 5).

**Embodiment 2**

In this embodiment, the fluorescence quantitative PCR method was used to detect the expression quantities of AQP2 in tumor cells and normal epithelial cells.

1. Materials

Head and neck squamous cell carcinoma cells SCC4 and human's normal oral epithelial cells HIOEC, kidney cancer cells 786-O and human's renal epithelial cells HEK293T, prostate cancer cells DU145 and human's normal prostate epithelial cells RWPE-1; the above cells were all purchased from U.S. ATCC Cell Repository.

2. Methods

2.1 Extraction of total RNA in tumor cells and normal epithelial cells

After the foregoing six types of cells were cultured in a 37 DEG C 5% $CO_2$ incubator until the density was 90%, they were digested and collected by trypsin, the cells were re-suspended in a culture solution and counted under a microscope, the concentration of the cells was adjusted to $5 \times 10^5$ cells/mL, and then the cell suspension after concentration adjustment was inoculated to 6-well plates, 2mL per well, and further cultured in the 37 DEG C 5% $CO_2$ incubator for 24 h.

Extract the total RNA in head and neck squamous cell carcinoma cells SCC4 and human's normal oral epithelial cells HIOEC, kidney cancer cells 786-O and human's renal epithelial cells HEK293T, prostate cancer cells DU145 and human's normal prostate epithelial cells RWPE-1, respectively according to the Trizol manual of Life Technologies, then quantify the purity and concentration of the extracted RNA by the NanoDrop ND-1000 nucleic acid quantifier, and ensure the integrity of the extracted RNA through quality inspection by agarose.

2.2 Synthesis of first-strand cDNA by reverse transcription of RNA

Use TaKaRa kit PrimeScriptTM RT kit with gDNA Eraser (Perfect Real Time) to reversely transcribe the extracted total RNA to synthesize cDNA. This kit contains gDNAEraser DNase and can effectively remove mingled genomic DNA.

2.3 Real-time quantitative PCR

Design specific primers according to the nucleotide sequences of AQP2 and GAPDH and use TaKaRa kit SYBR® Premix Ex Taq™ II (TliRNaseH Plus) to conduct PCR reaction. The forward primer and reverse primer of AQP2 are SEQ ID NO. 3 and SEQ ID NO. 4, and the forward primer and reverse primer of GAPDH are SEQ ID NO. 5 and SEQ ID NO. 6. The reaction system is shown in the table below:

Table 2. PCR reaction system

| Reagent | Dose ($\mu$L) |
| --- | --- |
| SYBR Premix Ex Taq II (TliRNaseH Plus) (2$\times$) | 12.5 |
| PCR Forward Primer (10 $\mu$M ) | 1 |
| PCR Reverse Primer (10 $\mu$M ) | 1 |
| DNA template (<100 $\mu$g) | 2 |
| Sterilized water | 8.5 |
| Total | 25 |

After mixing the above components evenly, carry out real-time quantitative PCR according to the following procedure: Initially denature at 95 DEG C for 30 s at 40 cycles; 95 DEG C for 5 s and 60 DEG C for 30 s.

Judge the specificity of the reaction according to the melting curve and calculate the mRNA expression quantity of AQP2 according to Formula $2^{-\triangle\triangle Ct}$. The result is shown in Fig. 6. Compared with the normal epithelial cells of human, the expression quantities of AQP2 in head and neck squamous cell carcinoma cells SCC4, kidney cancer cells 786-O and prostate cancer cells DU145 were reduced significantly, consistent with the analysis results of clinical samples.

## Embodiment 3

In this embodiment, the Western blot method was used to detect the expression quantities of AQP2 protein in tumor cells and normal epithelial cells.

Use trypsin to digest and collect the six types of cells in Embodiment 2 when the growth density reached 90%, use a culture solution to re-suspend the cells for multiplication culture, then collect the cells when the confluence was 80%, centrifuge, discard the supernatant, rinse with PBS twice and discard the supernatant. Add RIPA lysis buffer and lyse on ice for 20 min. Centrifuge at 12,000 g for 10 min and collect the supernatant. Add 1XSDS loading buffer, mix well by pipetting, then boil up and degenerate for 5 min. Separate total protein by 10% SDS-PAGE gel, then transfer it onto a PVDF membrane. block with 5% BSA at room temperature for 2 h, incubate with AQP2 antibody (abeam) overnight at 4 DEG C, and wash with TBST 3 times. Incubate with the secondary antibody at room temperature for 1 h and wash with TBST three times. Develop with an ECL supersensitive chemiluminescence solution, use the Tannon imaging system to form images and use GAPDH as an internal reference to compare the expression levels of AQP2 protein in different cells.

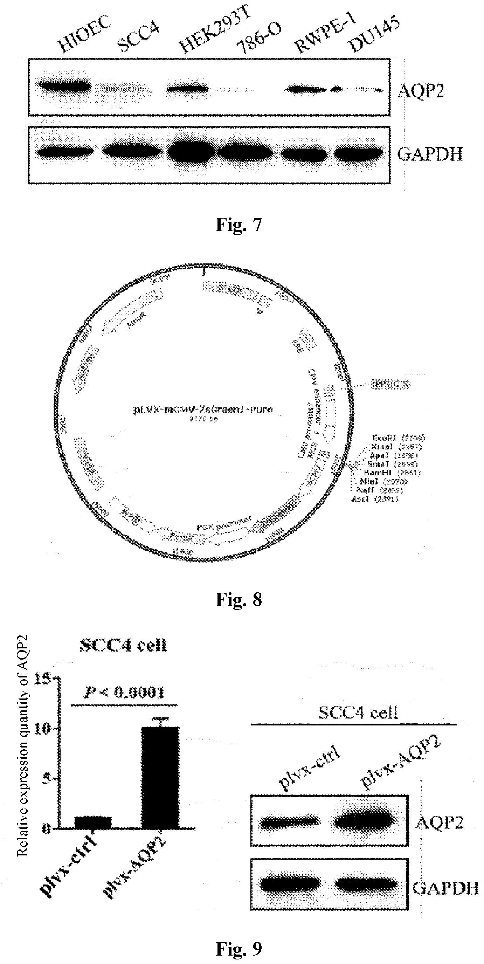

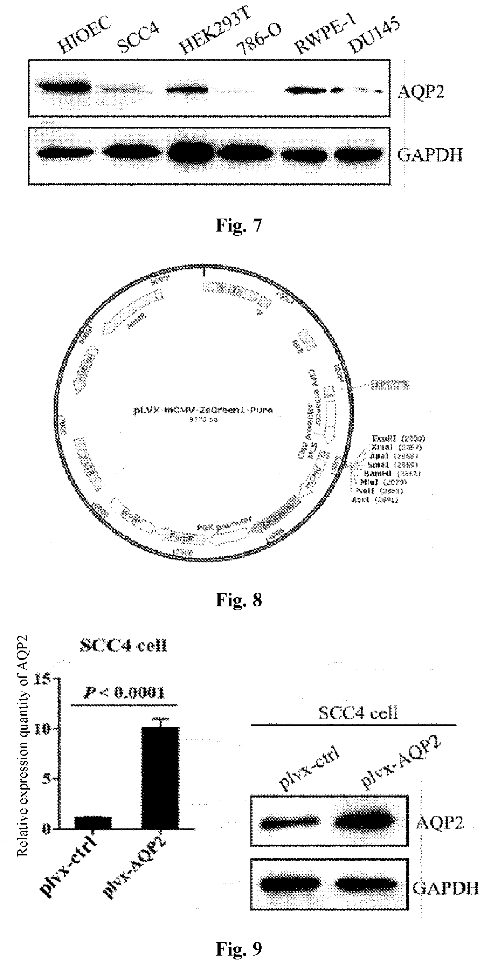

The results are shown in Fig. 7 and are consistent with AQP2 mRNA expression difference. The expression quantities of AQP2 protein in head and neck squamous cell carcinoma cells SCC4, kidney cancer cells 786-O and prostate cancer cells DU145 were reduced significantly.

## Embodiment 4

In this embodiment, the preparation of AQP2 overexpression vector and the detection of virus transfection efficiency were conducted.

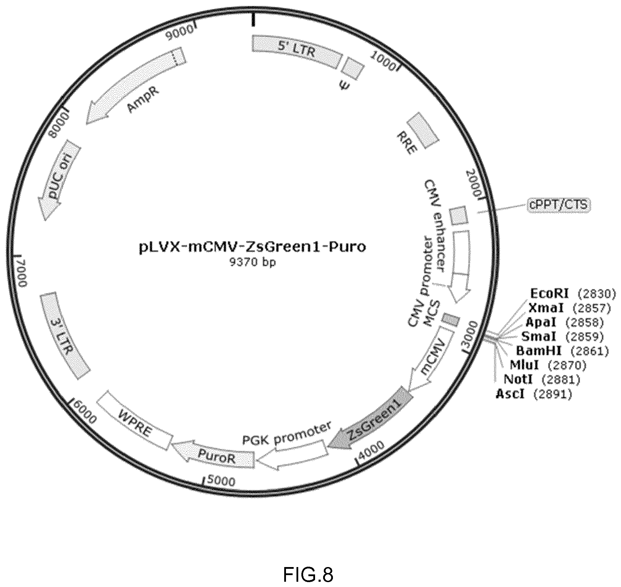

Synthesize full-length cDNA for AQP2 (see SEQ ID NO. 1 for the specific sequence) and introduce to plvx-CMV-ZsGreen 1 plasmid (see Fig. 8 for the atlas). Co-transfer the above plasmid, the packaging plasmid psPAX2 and the envelope plasmid pMD2.G into 293T cells to generate virus. After 48 hours of transfection, collect the viral supernatant of the cells and infect SCC4 head and neck squamous cell carcinoma cells, 786-Okidney cancer cells and DU145 prostate cancer cells, respectively. After 48 hours of infection, screen with puromycin for two weeks to obtain a cell line that stably promotes AQP2 gene expression. Collect the total RNA and total protein of the blank vectors and overexpression vectors of the three types of cells, and compare the expression changes of AQP2 gene and protein by qPCR (the specific method is the same as that in Embodiment 2) and Western blot method (the specific method is the same as that in Embodiment 3).

The results are shown in Fig. 9 to Fig. 11. Overexpression of AQP2 caused the expression quantities of AQP2 gene and protein in head and neck squamous cell carcinoma cells SCC4 (Fig. 9), kidney cancer cells 786-O (Fig. 10) and prostate cancer cells DU145 (Fig. 11) to increase significantly.

## Embodiment 5

This embodiment shows the effect of overexpression of AQP2 on the proliferation ability of human tumor cells.

Use trypsin to digest and collect head and neck squamous cell carcinoma cells SCC4, kidney cancer cells 786-O and prostate cancer cells DU145 after they stably transfected empty vectors and AQP2-overexpressed cells in a 37 DEG C 5% $CO_2$ incubator until the density was 90%, re-suspend the cells in a culture solution, count the cells under a microscope, adjust cell concentration to $3.0 \times 10^4$ cells/mL, inoculate the cell suspension to 96-well plates, 100$\mu$L per well, and culture in a 37 DEG C 5% $CO_2$ incubator for 24h, 48h and 72h, respectively. Add 20$\mu$L of 5 mg/mL MTT to each well of the 96-well plates, and continue to culture for 4 h. Suck away the culture medium and add 100 $\mu$L of DMSO for dissolution. Use ELIASA to detect absorbance at detection wavelength 570 nm and reference wavelength 630 nm and calculate proliferation inhibition (PI) according to the following formula:

$$PI\ (\%) = 1 - \text{drug group/negative group}$$

The test was independently repeated three times. The results obtained from the test were expressed with mean$\pm$SD, statistical $t$ test was done, the comparison of two or more groups of data adopted One-way ANOVA, statistical significance was expressed with value $P$, $P<0.05$ means significant difference, and $P<0.01$ means very significant difference.

The results are shown in Fig. 12 to Fig. 14. Compared with empty vector cells (plvx-crtl), the proliferation speed of the cells with overexpression of AQP2 (plvx-AQP2) (head and neck squamous cell carcinoma cells SCC4 (Fig. 12), kidney cancer cells 786-O (Fig. 14) and prostate cancer cells DU145 (Fig. 15)) was reduced obviously. It indicates that overexpression of AQP2 can significantly inhibit the proliferation of head and neck squamous cell carcinoma cells SCC4, kidney cancer cells 786-O and prostate cancer cells DU145 and further verifies the importance of AQP2 as a cancer suppressor gene.

## Embodiment 6

This embodiment shows the effect of overexpression of AQP2 on the migration ability of human tumor cells.

Inoculate head and neck squamous cell carcinoma cells SCC4, kidney cancer cells 786-O and prostate cancer cells DU145 to transwell cells, 100 $\mu$L per well, after they stably transfected empty vectors and AQP2-overexpressed cells, add 0.6 mL of complete medium containing 10% FBS to the transwell cells to stimulate cell migration, and culture in 5% $CO_2$ at 37 DEG C for 24 h. Discard the medium in the wells, fix with 90% alcohol at room temperature for 30 min, stain with 0.1% crystal violet at room temperature for 10 min, rinse with clear water, gently wipe off the non-migrated cells in the upper layer with a cotton swab, observe under a microscope and select four fields of view to take pictures and count. Calculate the migration inhibition rate (MIR) according to the following formula:

$$MIR(\%) = 1 - \frac{N_{test}}{N_{control}} \times 100\%$$

where $N_{test}$ is the number of migrated cells in the test group (plvx-AQP2) and $N_{control}$ is the number of migrated cells in the blank control group (plvx-ctrl). The test was independently repeated three times. The results obtained from the test were expressed with mean$\pm$SD, statistical t test was done, the comparison of two or more groups of data adopted One-way ANOVA, statistical significance was expressed with value $P$, $P<0.05$ means significant difference, and $P<0.01$ means very significant difference.

Table 3 Inhibitory effect of overexpression of AQP2 on the migration ability of human tumor cells

| Cell type | Group | No. of migrated cells (Mean ± SD) | MIR of cells (%) |
|---|---|---|---|
| SCC4 | plvx-ctrl | 661±48 | 0.00 |
| | plvx-AQP2 | 263±37 | 77.00%** |
| 786-O | plvx-ctrl | 727±41 | 0.00 |
| | plvx-AQP2 | 251±50 | 70.98%** |
| DU145 | plvx-ctrl | 707±39 | 0.00 |
| | plvx-AQP2 | 304±28 | 68.32%** |

In the table: * means $P<0.05$, **means $P<0.01$.

The results are as shown in Table 3. After AQP2 expression was upregulated, the migration abilities of head and neck squamous cell carcinoma cells SCC4, kidney cancer cells 786-O and prostate cancer cells DU145 were reduced obviously.

**Embodiment 7**

This embodiment shows the effect of overexpression of AQP2 on the in vivo growth of human tumor cells.

(1) Massively culture head and neck squamous cell carcinoma cells SCC4, kidney cancer cells 786-O and prostate cancer cells DU145 after they stably transfected empty vectors and AQP2-overexpressed cells, digest with a 0.25% pancreatin solution, centrifuge the cell suspension at 1,000 rpm for 5 min after termination of digestion, re-suspend the cells by a serum-free DMEM culture medium, then count the cells and adjust cell concentration to $5\times10^7$ cells/ml;

(2) Inoculate each nude mouse (female mice at the age of 4-6 weeks and with a weight of 14-16 g were ordered and adaptively reared for 1 week in an SPF animal breeding room) with 100 μl of the cell suspension of the corresponding group in the left armpit, and the number of cells injected is $5\times10^6$;

(3) After inoculation, the tumor growth at the inoculation sites of nude mice was closely observed. The volume of the transplanted tumor was measured and recorded every two days. The calculation formula of tumor volume (TV) is shown below:

$$TV=0.5\times a\times b^2$$

where, a is the length of the transplanted tumor (mm), and b is the width of the transplanted tumor (mm).

Compared with the empty vector control group (plvx-ctrl), the cells with overexpression of AQP2 (plvx-AQP2) (head and neck squamous cell carcinoma cells SCC4 (Fig. 15), kidney cancer cells 786-O (Fig. 16) and prostate cancer cells DU145 (Fig. 17)) showed a lower tumor growth rate and obviously reduced in-vivo tumorigenicity in nude mice, indicating that overexpression of AQP2 can inhibit the in vivo growth ability of malignant tumor cells.

**Claims**

1. Application of a transmembrane protein in the preparation of tumor treatment drugs or the use as a tumor marker, wherein the transmembrane protein is AQUAPORIN 2, and its amino acid sequence is shown in SEQ ID NO.2.

2. The application according to claim 1, wherein the tumor treatment is achieved by overexpressing the nucleotide sequence as shown in SEQ ID NO. 1 in the tumor.

3. A method for detecting tumor marker, wherein the detection method is any of a and b:

(a) detecting gene expression quantity of transmembrane protein AQUAPORIN 2 in tissue or cells by the PCR method;

(b) detecting gene expression quantity of transmembrane protein AQUAPORIN 2 in tissue or cells by the Western blot method.

**4.** The detection method according to claim 3, wherein the PCR method includes a specific primer pair designed for SEQ ID NO. 1 sequence.

**5.** The detection method according to claim 4, wherein the specific primer pair includes SEQ ID NO. 3 and SEQ ID NO. 4.

**6.** The application according to claim 1 or 2 and the detection method according to claims 3 to 5, wherein the tumors include head and neck squamous cell carcinoma, kidney cancer and prostate cancer.

**7.** A recombinant vector, wherein the recombinant vector contains a nucleotide sequence as shown in SEQ ID NO. 1.

**Fig. 1**

**Fig. 2**

**Fig. 3**

**Fig. 4**

**Fig. 5**

**Fig. 6**

**Fig. 7**

**Fig. 8**

**Fig. 9**

FIG.8

**Fig. 7**

**Fig. 8**

**Fig. 9**

**Fig. 10**

**Fig. 11**

**Fig. 12**

**786-O cell**

Fig. 13

**DU145 cell**

Fig. 14

## SCC4 cell

**Fig. 15**

## 786-O cell

**Fig. 16**

Fig. 17